# EUROPEAN PATENT SPECIFICATION

(11) **EP 4 518 745 B1**
(45) Date of publication and mention of the grant of the patent: **26.11.2025**
(21) Application number: 23725796.9
(22) Date of filing: 27.04.2023
(51) Int. Cl.: A61B 5/00, A61B 5/07, A61M 31/00

(54) **DEGRADABLE DEVICE FOR THE PASSIVE AND MONITORED RELEASE OF A SUBSTANCE**
ABBAUBARE VORRICHTUNG ZUR PASSIVEN ÜBERWACHTEN FREISETZUNG EINER SUBSTANZ
DISPOSITIF DÉGRADABLE POUR LIBÉRATION PASSIVE ET SURVEILLÉE D'UNE SUBSTANCE

(30) Priority: 03.05.2022 IT 202200008993
(43) Date of publication of application: 12.03.2025
(73) Proprietor: Fondazione Istituto Italiano di Tecnologia, 16163 Genova (GE) (IT)
(72) Inventor: LAMANNA, Leonardo, 16163 Genova GE (IT); CATALDI, Pietro, 16163 Genova GE (IT); CAIRONI, Mario, 16163 Genova GE (IT)
(74) Representative: Perani & Partners S.p.A.
(86) International application number: PCT/IB2023/054352
(87) International publication number: WO 2023/214260

(56) References cited:
- US-A1- 2004 253 304
- US-A1- 2008 284 599
- US-A1- 2012 062 379
- US-A1- 2016 135 919
- US-A1- 2018 132 784

## Description

The present invention relates to a degradable device for the passive and traceable release of a substance in a solution.

The present invention relates to a system provided with said device for monitoring the passive release of a substance into a solution.

The present invention also relates to a non-medical method employing the aforesaid device for monitoring the passive release of a substance into a solution.

The present invention also relates to an oral pill containing said device.

### Description of the prior art

An accurate and prolonged drug therapy is fundamental for disease control and/or for healing for many diseases, such as diabetes, hypertension, psychiatric diseases. These therapies are based on two main requirements: a proper pharmacological adherence and a controlled drug release system. Pharmacological adherence describes the degree to which a patient correctly follows a prescription. In medical practice, the patient's pharmacological non-compliance is a major obstacle to the effective delivery of health care.

It is known in the art to realize ingestible electronic systems such as endo-radioprobes capable of detecting the pH. These are user ingestible devices configured to provide a measurement of different physiological parameters of the gastrointestinal system (e.g. pH). An ingestible micro-sensor with integrated microelectronic circuit (e.g. CMOS), designed to be incorporated into a tablet for monitoring drug adherence is also known.

On the other hand, the controlled drug release systems allow the drug dosages to be adjusted at specific rates. This mode of delivery maintains the concentration of the drug at an optimal therapeutic level, maximizing its effectiveness, reducing the amount and the frequency of administration of the drugs and their side effects. In the prior art, there is a need to develop new materials/scaffolds to enhance the pharmacological activities of the drugs and to overcome problems of solubility, aggregation, bioavailability, biodistribution, selectivity and to reduce the side effects of the therapeutic drugs.

Document US2008284599A1 describes a pharmaceutical-computer system and, in particular, a method for the automatic identification of a pharmaceutical material, ensuring that a patient correctly follows the prescribed therapy. The disclosed embodiments comprise compositions with an active agent, an identifier and a pharmaceutically acceptable carrier. An ingestible pill is identifiable via an integrated electronic microchip, which transmits a signal when it is dissolved in a solution, such as the stomach fluids. The transmitted signal is received by another device within or near the body. In addition, it can be verified that the pill has reached the stomach and is about to dissolve.

Document US2016135919A1 discloses a method for managing the ingestion state of medicaments, and a device for managing the ingestion state of medicaments. An ingestible drug comprises a medicament soluble in gastric juices and a chip capable of generating energy upon exposure with gastric juices. The chip is able to determine the date and the time when the patient digested the medicament.

### Problem of the prior art

In the prior art, the ingestible electronic devices have achieved excellent performance by exploiting standard electronic components. However, the use of materials of conventional electronics that are toxic to humans and/or to the environment, the need for medical supervision during the treatment and the *ex post-*recovery put strong limitations on the use of such devices. In fact, the risks of retention and obstruction are considerable. Therefore, in a perspective of large-scale application, this would require a frequent and therefore unsustainable recourse to surgery for the removal of such devices from the body of the patients.

In addition, the known drug administration systems lack real-time monitoring of the drug release, which is only evaluated retroactively through blood, tissue, and stool analyses. Such a procedure is expensive and provides only statistical results on the release of drugs in the patients taking drugs every day. In fact, the release profiles vary depending on metabolism, diet, and proper drug intake.

One of the main limitations in the development of such safe and self-administrable devices is an adequate detection and communication system compatible with the edible electronics. In fact, the standard wireless communication platforms such as Bluetooth, Zigbee or Wi-Fi require complex logic circuits, such as the aforementioned CMOS technology, as well as low impedance conductors for the implementation of antennas and relatively high-power density in the order of hundreds nJ/bit. These conditions are not currently replaceable with edible electronic components.

### SUMMARY OF THE INVENTION

Aim of the present invention is to realize a device capable of providing real-time information on the release of a substance.

More in detail, aim of the present invention is to realize an ingestible, in particular edible, digestible and metabolizable device capable of providing real-time information on the release of a drug, permitting an instantaneous picture of when, where and how much drug has been released.

The stated technical task and specified objects are substantially achieved by a device comprising the technical features presented in the claim 1.

### Advantages of the invention

Thanks to one embodiment, it is possible to obtain a device compatible with the edible electronics, which allows a real-time monitoring of the release of a substance contained therein.

Thanks to one embodiment, it is possible to realize a device for monitoring the release of a drug.

Thanks to one embodiment, it is possible to realize a system that allows to monitor the release of a drug and the pharmacological adherence.

Thanks to one embodiment it is possible to realize a non-medical method for monitoring the release of a substance, and in particular of a drug, in a liquid environment with ionic conductivity, and which is in particular aqueous.

Thanks to a further embodiment, it is possible to realize a pill containing the device of the invention for the release of a substance, and in particular of a drug, in the gastro-intestinal tract.

### BRIEF DESCRIPTION OF THE DRA WINGS

The characteristics and the advantages of the present invention will be apparent from the following detailed description of possible practical embodiments, illustrated by way of non-limiting example in the set of drawings, in which:
- Figure 1 shows a device and relative system according to the present invention,
- Figure 2 shows a system and a device according to the present invention following a process of degradation of part of the same device,
- Figure 3 schematically shows in a) an example of application of the system of the present invention with a pill incorporating the device of Figures 1 and 2, in b) the trend of the signal emitted by the device as a function of the degradation of part of the same device, and in c) a schematic example of a pill comprising the device of the invention,

- Figure 4 shows some steps of the method for realizing the pill of Figure 3c,
- Figure 5 shows schematically in a) a first mode of functioning of the system of the invention with the device in the condition of Figure 2, and in b) a second mode of functioning of the system of the invention with the device in the condition of Figure 1,
- Figure 6 shows the wiring diagram describing the electrical functionality of the device (1),
- Figure 7 shows in a) the degradation over time of the device of the invention in the intestinal tract, and in b) the trend of the electrical signal emitted by the device during degradation,
- Figure 8 shows an example of application of the device of Figure 1 and relative system used for the monitored release of a substance in an aquarium.

The device illustrated in the accompanying figures is to be understood as schematically represented, not necessarily to scale and not necessarily with the proportions represented between the various constituent elements.

### DETAILED DESCRIPTION

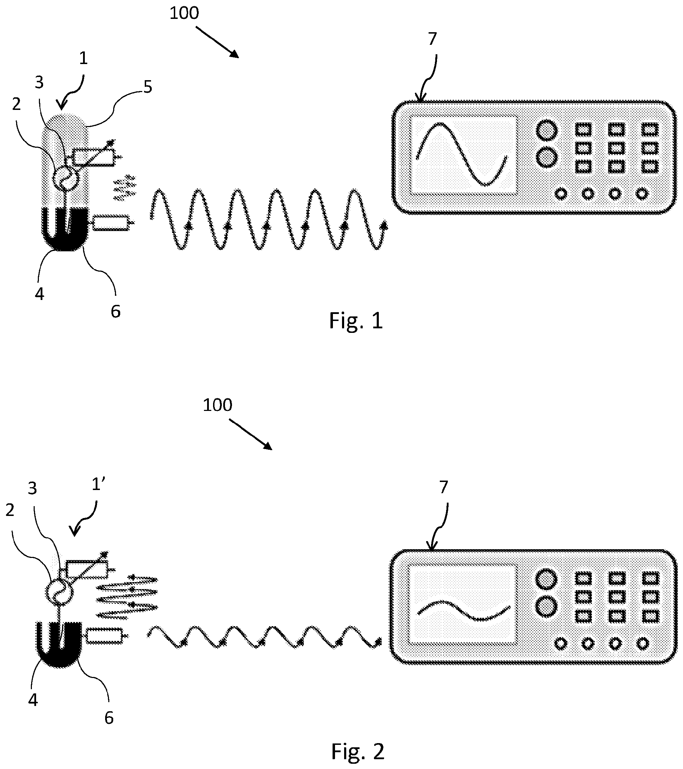

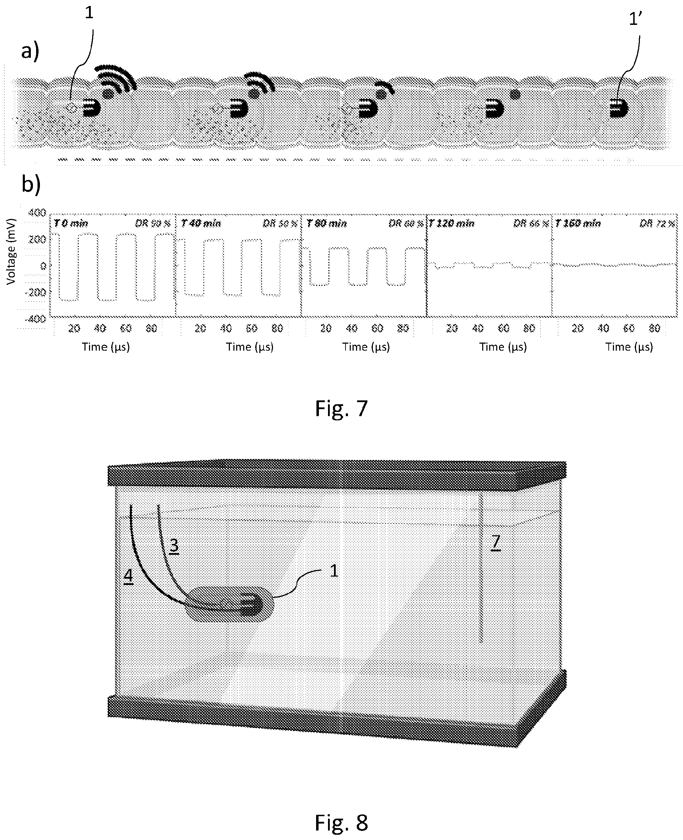

The present invention relates to a device 1, illustrated in Figure 1, for the passive and traceable release of a substance in a solution outside the device 1. Within the context of the present invention, the preferred embodiment provides that the outer solution, i.e. the one in which the device 1 is intended to be immersed when in use, is made in an aqueous environment or, if different, in any case provided with ionic conductivity. The device can for example be used in order to release a substance in an aquarium (Fig. 7), monitoring its degradation over time until total release of the substance, as shown in Fig. 2 where the fully degraded device 1' is reported. The device is applicable, for example, for the controlled release of disinfectants and/or dyes in a swimming pool, monitoring their concentration. This example of application will be detailed later in this description. According to a further example of use, the device is applicable in a field or in a hydroponic type crop, for monitoring the release of fertilizers. The device is also applicable for the controlled release of chelating and/or disinfecting agents in sludge or wastewater.

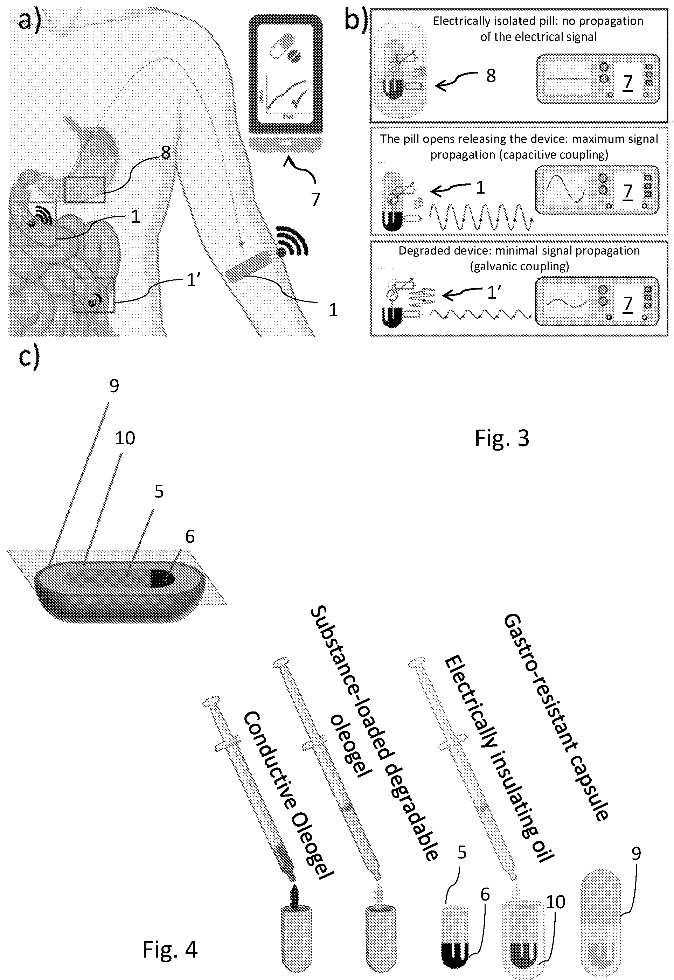

A further example of application, which will be discussed in more detail hereinafter, is shown in Figures 3 and 7, wherein the device 1 is used for the controlled release of a substance in the gastro-intestinal tract.

The device 1 comprises a transmitter 2 configured to generate an electrical signal and provided with a first electrode 3 and with a second electrode 4. In other words, the first electrode 3 and the second electrode 4 correspond to the two terminals of the transmitter 2. In the preferred embodiment of the invention, the transmitter 2 is an oscillator configured to generate an alternating current signal.

The device 1 further comprises a battery (not shown in the accompanying figures) adapted to electrically power the transmitter 2.

The device 1 comprises a passive release component 5 made at least in part of electrically insulating material and comprising the substance to be released. The passive release component 5 is degradable in a solution in order to release the substance over time. In addition, the passive release component 5 incorporates the first electrode 3, so as to isolate it electrically at least partially, preferably totally from the outside, i.e., from the solution in which the device is intended to be immersed to allow the degradation of the passive release component 5 and consequent release of the substance (Figure 2). In this regard, it is worth noting that by completely degraded device 1' is meant the device in which the passive release component 5 is completely degraded totally exposing the first electrode 3 to the outer solution. Preferably, the passive release component 5 can be made of suitably selected material in order to release the substance contained therein with the desired degradation mode in the target solution. The target solution is clearly defined by the application in which the device is intended to be used. For example, the target solution coincides with the environment of the human body in which the device is intended to be conveyed for the release of the substance, such as the intestinal fluid in the preferred application of the invention illustrated in the accompanying figures and described hereinafter.

The device 1 comprises a stable component 6, that is, it does not degrade when immersed in the target, electrically conductive solution that incorporates the second electrode 4. In this way, the electrical signal communication between the second electrode 4 and the solution in which the device 1 in use is intended to be immersed is always allowed by the stable component 6.

The substance to be released can be of any type depending on the application of the device 1. For example, the substance may comprise salts to be released into an aquarium, or disinfectants or anti-algae substances for the treatment of the water of a swimming pool. As will be seen below, in case of use of edible components, the device 1 can be employed for example as a tablet or incorporated into a pill provided with a gastro-resistant capsule, in which the substance could comprise a food supplement or an active ingredient.

According to a preferred embodiment, the transmitter 2 is incorporated into the passive release component 5. However, the preferred solution of the invention provides for the transmitter 2 to be incorporated or attached externally to the stable component 6.

In accordance with a preferred solution of the invention, the transmitter 2 and the battery and the stable component 6 are ingestible and more preferably edible electronic components.

Within the context of the present invention, by an ingestible electronic device/component is meant a device with conventional generic electronic components, based on silicon or technologies that are not necessarily compatible with the ingestion. This property is guaranteed by an appropriate isolation that does not allow the direct contact between the electronics and the human body, for example with a rigid capsule that integrates microcontrollers, microchips, batteries or LEDs, which will be expelled from the body just as it was ingested.

Within the context of the present invention, by an edible (or eatable) electronic device/component is meant a device made with only eatable components and materials, that is, which can be ingested in total safety and which are degraded within the human body, digested and/or metabolized depending on the specific materials used.

Preferably, the passive release component 5 is a matrix of degradable material, and more preferably of digestible material, in which the substance is contained. Always preferably, the passive release component 5 comprises an electrically insulating oleogel matrix (in short, oleogel). Still preferably, the stable component 6 comprises an electrically conductive oleogel matrix.

According to a preferred embodiment of the invention, the electrically insulating oleogel matrix comprises beeswax and sunflower oil. Preferably, the electrically conductive oleogel matrix comprises beeswax, sunflower oil and activated carbon. Advantageously, in the preferred embodiment just described the device 1 envisages using new materials composed of food and food derivatives. In the preferred embodiment, the conductive oleogel has a resistivity of about 100 Ω cm, and comprises beeswax and sunflower oil with weight ratio of 25:75 and is loaded with 40% by weight of activated carbon. Instead, the degradable/digestible matrix for the encapsulation of the drug is an insulating oleogel matrix made with the same components as the conductive oleogel, without the activated carbon. Preferably, hydroxyethylcellulose (HEC) is added as a filler in the insulating oleogel in order to trigger the disintegration of the device 1 in the human intestine. It is worth noting that HEC is a hydrophilic natural polysaccharide. In this preferred embodiment, the liquid environment results in the degradation of the component 5 thanks to the disintegrating action of hydroxyethylcellulose.

In accordance with a solution of the invention, the substance comprises at least one food supplement.

In accordance with a solution of the invention, the substance comprises at least one active ingredient. Preferably, at least one active ingredient comprises metformin. Preferably, the insulating oleogel is loaded with 10% by weight of metformin. In other words, the device 1 in which the passive release component 5 comprises one or more pharmacological active ingredients finds application in the medical field.

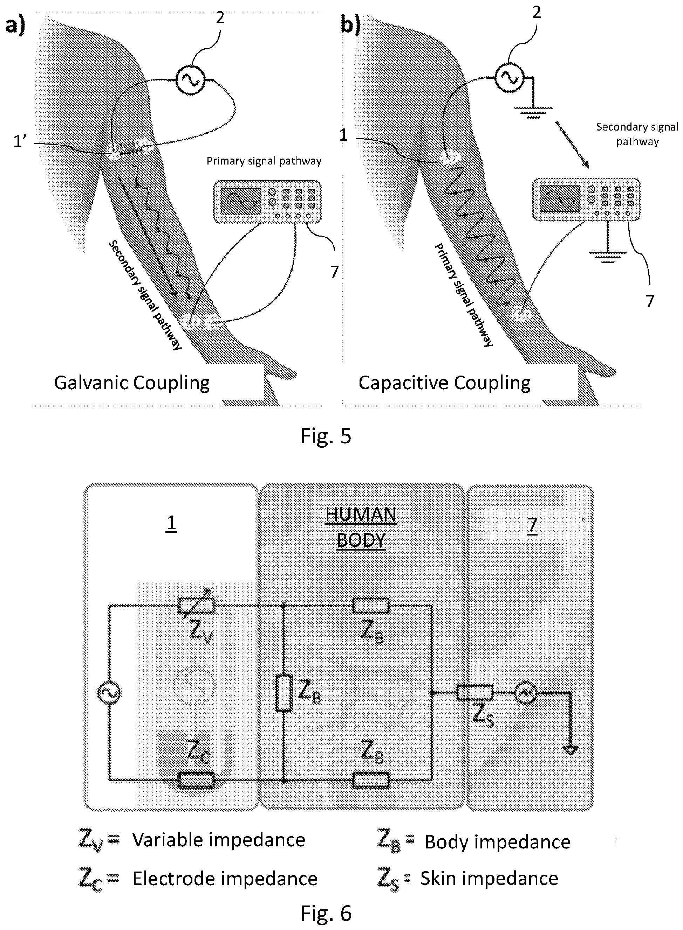

With reference to the example of application referred to in Figures 3, 5 and 6, it is worth noting that the device 1 of the invention exploits intra body communication, in short IBC. IBC is a wireless, bio-inspired (e.g., by the nervous system) communication platform that exploits the body's intrinsic ionic conductivity to propagate a signal, generating a so-called wireless body area network (in short WBAN). IBC is therefore applicable in aqueous solutions of any type. More generally, this is therefore ionic conductivity mediated communication. Two main coupling techniques are possible for IBC: galvanic and capacitive. The galvanic coupling has four electrodes in contact with the human body, two for transmission and two for reception (Fig. 5a). In this system, a small current is injected into the human body. The capacitive coupling, on the other hand, exploits only two electrodes in contact with the human body, where an electrical potential is generated between the signal (contact) and ground (directed towards the external environment) electrodes of the transmitter 2. The electrical potential is detected by the receiver 7, which in turn has a pair of signal and ground electrodes in the same configuration as the transmitter 2 (Fig. 5b). The electrical potential induced by the signal electrode of the transmitter 2 is coupled to the human body and is detected by the receiver 7 through it, with the advantage of a large part of the signal being confined to the human body, minimizing the required transmission power. The IBC technology has the advantage of low power consumption (~10 pJ/bit) and the possibility to use high impedance conductors, compatible with the batteries and the edible conductors.

The invention allows to realize an ingestible, and preferably edible, device 1 with electrically monitored passive drug release by exploiting a passage between the capacitive and galvanic coupling modes of the IBC. As will be described in more detail hereinafter, an application of the invention consists of a pill 8 containing the device 1, which as said comprises a conductive composite and of an insulating matrix loaded with degradable/digestible drug. Each part is connected to a terminal of an AC signal generator/transmitter 2. The drug used is metformin, which is a hypoglycemic agent for the management of type 2 diabetes.

Advantageously, the device of the invention is ingestible, preferably edible, ecological, economical in the components and in its realization, safe and self-administrable.

Advantageously, the device 1 is safe for monitoring the therapeutic adherence and the controlled release of the drug.

Optionally, the device 1 may comprise a protective outer coating (not illustrated), which is degradable in the solution in which it is immersed in order to activate the device 1. It is worth noting that the outer coating is completely optional.

The present invention also relates to a system 100 for monitoring the passive release of a substance into a solution. The system 100 comprises a device 1 according to the invention and a receiver 7 configured to detect the electrical signal generated by the transmitter 2 after propagating through an aqueous outer medium via IBC. In this regard, it should be noted that in the examples of Figures 3, 5 and 6 the aqueous outer medium is the human body with its impedance, while in the example of Figure 8 the aqueous outer medium is the solution that fills an aquarium. In detail, with particular reference to Figures 1, 2, 3b, 5 and 7 it is pointed out that the device 1 and the receiver operate according to a capacitive coupling when the passive release component 5 is intact and the first electrode 3 is electrically insulated. Whereas, the electrical signal generated by the transmitter 2 is gradually reduced as the passive release component 5 degrades until it reaches a minimum or negligible intensity value (i.e. a much lower value than the starting signal, a measurable value or which is even potentially likely to be confused with the electronic noise), when the passive release component 5 is completely degraded (i.e. completely degraded device 1') exposing the first electrode 3 to the surrounding solution. In this configuration, the device 1 and the receiver 7 operate according to a galvanic coupling in which the first electrode 3 and the second electrode 4 are short-circuited (circuit diagram Figure 6).

The present invention further relates to a method for monitoring the passive release of a substance into a solution by means of the system 100 of the invention. It is worth noting that the method for monitoring the passive release of a substance into a solution by means of a system 100 is employed for non-medical use. In other words, the method is employed for purposes other than the diagnostic and/or therapeutic ones on the human or animal body.

The method comprises the step of immersing the device 1 into a solution. The method then comprises the step of detecting with the receiver 7 the trend over time of the electrical signal generated by the transmitter 2 of the device 1 and propagated through an aqueous medium that is at least partially electrically conductive. In addition, the method envisages the step of associating a reduction in the intensity of the electrical signal detected by the receiver 7 with the progress of the degradation state of the passive release component 5 with gradual release of the substance. Finally, the method envisages identifying the total degradation of the passive release component 5 when the detected electrical signal is minimal or substantially nil (i.e. when it reaches minimal or negligible intensity, or in any case lower than a threshold value). It is worth noting that this last step consists of detecting the switching from the capacitive coupling to the galvanic coupling (Figure 5).

The present disclosure also relates to an unclaimed method for monitoring the passive release of a substance, and in particular of a drug or active ingredient, in a solution by means of the system 100 of the invention. In the preferred embodiment, the device 1 forming part of the system 100 is part of a pill 8, which will be described in detail hereinafter. This method can be used to monitor the pharmacological adherence of a patient to a given drug, as well as the timing of the degradation until total release of the drug. The method comprises the step of immersing the device 1 into a solution, which in this embodiment is identifiable in an environment of the human body, such as the intestinal fluid. The method then comprises the step of detecting with the receiver 7 the trend over time of the electrical signal generated by the transmitter 2 of the device 1 and propagated through an aqueous medium that is at least partially electrically conductive which, specifically, is the human body or a circumscribed part thereof. The electrical signal generated by the transmitter 2 therefore propagates through the human body until it is detected by the receiver 7, exploiting the intra body communication IBC (Figures 3 and 5). In addition, the method envisages the step of associating a reduction in the intensity of the electrical signal detected by the receiver 7 with the progress of the degradation state of the passive release component 5 with gradual release of the drug or active ingredient. Finally, the method envisages identifying the total degradation of the passive release component 5 when the detected electrical signal is minimal or substantially nil (i.e. of minimal or negligible intensity, or in any case lower than a threshold value). It is worth noting that this last step consists of detecting the switching from the capacitive coupling to the galvanic coupling (Figure 5). As will be described in detail below, the method with the use of a pill 8 allows to identify the position of the pill 8 when it reaches the intestine as the dissolution of the gastro-resistant shell of the pill allows the propagation of the IBC electrical signal generated by the transmitter 2, which can therefore be detected by the receiver 7.

The present invention also relates to a pill 8 for the passive and traceable release of a substance in the intestine. The pill 8 comprises the device 1 and an outer gastro-resistant capsule 9, preferably electrically insulating, which encloses the device 1.

Preferably, the pill 8 comprises an electrically insulating oil 10 enclosed in the outer gastro-resistant capsule 9, in which the device 1 is immersed in the electrically insulating oil 10. The structure of the pill 8 ensures that the transmission of the coupled capacitive IBC electrical signal only begins in the intestine along with the release of the drug, after the dissolution of the gastro-resistant protective shell.

It should be specified that the pill 8, which comprises the device 1, can be used in the system 100 in the manners described above.

After ingestion, the pill 8 passes through the stomach intact and reaches the intestine protected by a layer of electrically insulating oil 10, sealed in the outer gastro-resistant capsule 9, which prevents both the transmission of the signal and the release of the drug. Once the pill 8 reaches the intestine, the environmental/pH change triggers the activation of the device 1, by dissolving the outer gastro-resistant capsule 9. The stable conductive component 6 of the device 1 comes into contact with the intestinal fluid and a coupled capacitive IBC signal is transmitted, which allows to determine the time elapsed from the ingestion of the pill 8 to its arrival in the intestine, corresponding to the beginning of the release of the drug. The drug-filled composite, of which the passive release component 5 is composed, is designed to dissolve in the intestinal fluid. This dissolution leads to a gradual transition from the capacitive to the galvanic coupling with a consequent reduction in the transmission amplitude of the electrical signal. The transmitted signal reaches its minimum by complete dissolution of the device 1, which "short-circuits" the AC signal, passing to a galvanic IBC coupling. Advantageously, the pill 8 allows to passively monitor the administration of drugs, providing real-time information on pharmacological adherence and drug release.

In the preferred embodiment of the invention, the device 1 allows the real-time monitoring of the pharmacological adherence and of the intestinal release of metformin. The core of the pill 8, formed by the device 1, comprises a stable conductive part and a degradable insulating part filled with metformin. Each part is connected to a terminal of an AC signal generator, i.e. the transmitter 2. The capacitive-IBC transmission signal is passively activated upon reaching the intestine, where it begins to release the drug and is read wirelessly by a receiver (Fig. 1, 2, 3 and 7). As mentioned above, the change in the IBC coupling is exploited in the invention as a detection method for monitoring the drug release. In particular, together with the release of the drug, there is a gradual attenuation of the reading signal, corresponding to a gradual switching between the capacitive coupling and the galvanic coupling of the IBC. The capacitive coupling-based IBC has a lower signal attenuation than the galvanic coupling. In fact, the galvanic IBC has a primary signal pathway between the transmission electrodes and only a small secondary current passes through the receiver (Fig. 5). As a result, the body effectively short-circuits the transmission signal, leading to a greater attenuation of the signal transmission. In contrast, the capacitive IBC has a primary signal pathway through the human body and only a small part of this signal is lost to the outer mass (secondary pathway).

Figure 6 reports the circuit model of the IBC coupling mechanism exploited by device 1, containing signal generator/transmitter 2, device 1, body, skin and receiver 7. The pill 8 has a constant impedance (conductive oleogel, Z_{C}) and a variable impedance (degradable/digestible drug filled matrix, Z_{V}). The other impedances represent the inner impedance of the body (Z_{B}) and the skin impedance (Z_{S}) which are constant. The decrease of Z_{V} due to the degradation of the device 1 leads to a "short circuit" of the signal, allowing the passage from a capacitive to a galvanic IBC coupling regime. This results in a progressive reduction in the reading signal (Fig. 7a and 7b).

Figure 4 schematically illustrates some steps of the method for making the pill 8. In particular, the method for realizing the pill 8 according to the preferred embodiment of the invention envisages filling a mould firstly with the stable conductive oleogel, followed by the electrically insulating degradable oleogel loaded with the substance, for example metformin. The addition of the edible electronic components that are incorporated into the oleogel completes the device 1. Thereafter, the device is arranged within the shell of an outer gastrointestinal capsule 9. The capsule is then filled with electrically insulating oil 10 and finally closed.

According to a use described above, the device 1 is employable in order to release in a controlled manner a substance into an aquarium. In accordance with a preferred solution of the invention, the transmitter 2 and the battery and the stable component 6 are edible and environmentally friendly electronic components. In other words, they can be ingested in total safety and are degraded within the body of animals.

In use, the method comprises the step of immersing the device 1 into a solution, for example the water contained in an aquarium. The method then comprises the step of detecting with the receiver 7 the trend over time of the electrical signal generated by the transmitter 2.

The present invention also relates to a method for monitoring the passive release of a substance, and in particular of a substance within an aquatic environment. This method can be used to monitor the distribution of a substance such as for example a disinfectant.

Preferably, an outer coating is employable, for the passive and traceable release of a substance within, for example salts, an aquatic environment such as a marine aquarium. Advantageously, the outer coating, which is optional, prevents the device 1 from being damaged prematurely for example by fish or by accidental impacts.

The environmental/pH change triggers the activation of the device 1, by dissolving the outer coating.
The project from which the present patent application derives has received funding from the research and innovation program of the European Union Horizon 2020, contract no. 864299.

## Claims

1. Device (1) for the passive and traceable release of a substance in a solution, comprising:
- a transmitter (2) configured to generate an electrical signal and provided with a first electrode (3) and with a second electrode (4);
- a battery adapted to electrically power the transmitter (2);
- a passive release component (5) comprising said substance and which is degradable in a solution in order to release said substance,
wherein
- the first electrode (3) is incorporated into the passive release component (5),
- the passive release component (5) is made at least in part of electrically insulating material;
**characterized in that** it comprises a stable electrically conductive component (6) into which the second electrode (4) is incorporated.

2. Device (1) according to claim 1, wherein
- the transmitter (2) is an oscillator.

3. Device (1) according to any one of the preceding claims, wherein
- the transmitter (2), the battery and the stable component (6) are ingestible and preferably edible electronic components, and
- the passive release component (5) is a matrix of digestible material in which the substance is contained.

4. Device (1) according to claim 3, wherein
- the passive release component (5) comprises an electrically insulating oleogel matrix,
- the stable component (6) comprises an electrically conductive oleogel matrix.

5. Device (1) according to claim 4, wherein
- the electrically insulating oleogel matrix comprises beeswax and sunflower oil,
- the electrically conductive oelogel matrix comprises beeswax, sunflower oil and activated carbon.

6. Device (1) according to any one of claims 3 to 5, wherein
- the substance comprises at least one active ingredient.

7. Device (1) according to claim 6, wherein
- the at least one active ingredient comprises metformin.

8. System (100) for monitoring the passive release of a substance into a solution, comprising:
- a device (1) according to any of claims 1 to 7;
- a receiver (7) configured to detect the electrical signal generated by the transmitter (2) and propagated through an aqueous outer medium by exploiting the intra body communication and the coupling switching of the intra body communication.

9. Method for monitoring the passive release of a substance into a solution by means of a system (100) according to claim 8 wherein the device (1) is one according to any of claims 1 to 5, for non-medical use, comprising the steps of:
- immersing the device (1) into a solution;
- detecting with the receiver (7) the trend over time of the electrical signal generated by the transmitter (2) of the device (1) and propagated through an aqueous medium;
- associating a reduction in the intensity of the electrical signal detected by the receiver (7) with the progress of the degradation state of the passive release component (5) with release of the substance.

10. Method according to claim 9, comprising the step of:
- identifying the total degradation of the passive release component (5) when the detected electrical signal is of minimal or negligible intensity.

11. Pill (8) for the passive and traceable release of a substance in the intestine, comprising;
- a device (1) according to any one of claims 3 to 7;
- an outer gastro-resistant capsule (9) which encloses the device (1).

12. Pill (8) according to claim 10, comprising;
- an electrically insulating oil (10) enclosed in the outer capsule (9), the device (1) being immersed in the electrically insulating oil (10).

## Patentansprüche

1. Vorrichtung (1) zur passiven und rückverfolgbaren Freisetzung einer Substanz in einer Lösung, umfassend:
- einen Sender (2), der dazu konfiguriert ist, ein elektrisches Signal zu erzeugen und mit einer ersten Elektrode (3) und einer zweiten Elektrode (4) versehen ist;
- eine Batterie, die dazu ausgelegt ist, den Sender (2) elektrisch mit Strom zu versorgen;
- eine passive Freisetzungskomponente (5), die die Substanz umfasst und die in einer Lösung abbaubar ist, um die Substanz freizusetzen,
wobei
- die erste Elektrode (3) in die passive Freisetzungskomponente (5) integriert ist,
- die passive Freisetzungskomponente (5) zumindest teilweise aus elektrisch isolierendem Material besteht;
**dadurch gekennzeichnet, dass** sie eine stabile elektrisch leitfähige Komponente (6) umfasst, in die die zweite Elektrode (4) integriert ist.

2. Vorrichtung (1) nach Anspruch 1, wobei
- der Sender (2) ein Oszillator ist.

3. Vorrichtung (1) nach einem der vorhergehenden Ansprüche, wobei
- der Sender (2), die Batterie und die stabile Komponente (6) einnehmbare und vorzugsweise essbare elektronische Komponenten sind und
- die passive Freisetzungskomponente (5) eine Matrix aus verdaulichem Material ist, in der die Substanz enthalten ist.

4. Vorrichtung (1) nach Anspruch 3, wobei
- die passive Freisetzungskomponente (5) eine elektrisch isolierende Oleogelmatrix umfasst,
- die stabile Komponente (6) eine elektrisch leitfähige Oleogelmatrix umfasst.

5. Vorrichtung (1) nach Anspruch 4, wobei
- die elektrisch isolierende Oleogelmatrix Bienenwachs und Sonnenblumenöl umfasst,
- die elektrisch leitfähige Oleogelmatrix Bienenwachs, Sonnenblumenöl und Aktivkohle umfasst.

6. Vorrichtung (1) nach einem der Ansprüche 3 bis 5, wobei
- die Substanz mindestens einen Wirkstoff umfasst.

7. Vorrichtung (1) nach Anspruch 6, wobei
- der mindestens eine Wirkstoff Metformin umfasst.

8. System (100) zur Überwachung der passiven Freisetzung einer Substanz in eine Lösung, umfassend:
- eine Vorrichtung (1) nach einem der Ansprüche 1 bis 7;
- einen Empfänger (7), der dazu konfiguriert ist, das von dem Sender (2) erzeugte und sich durch ein wässriges äußeres Medium ausbreitende elektrische Signal unter Ausnutzung der körpereigenen Kommunikation und des Kopplungsschaltens der körpereigenen Kommunikation zu erfassen.

9. Verfahren zur Überwachung der passiven Freisetzung einer Substanz in eine Lösung mittels eines Systems (100) nach Anspruch 8, wobei es sich bei der Vorrichtung (1) um eine solche nach einem der Ansprüche 1 bis 5 handelt, zur nicht-medizinischen Verwendung, umfassend die Schritte:
- Eintauchen der Vorrichtung (1) in eine Lösung;
- Erfassen mit dem Empfänger (7) des Verlaufs über die Zeit des vom Sender (2) der Vorrichtung (1) erzeugten und sich durch ein wässriges Medium ausbreitenden elektrischen Signals;
- Verknüpfen einer Verringerung der Intensität des vom Empfänger (7) erfassten elektrischen Signals mit dem Fortschreiten des Abbauzustands der passiven Freisetzungskomponente (5) mit der Freisetzung der Substanz.

10. Verfahren nach Anspruch 9, umfassend den Schritt:
- Ermitteln des Gesamtabbaus der passiven Freisetzungskomponente (5), wenn das erfasste elektrische Signal von minimaler oder vernachlässigbarer Intensität ist.

11. Pille (8) zur passiven und rückverfolgbaren Freisetzung einer Substanz in den Darm, umfassend:
- eine Vorrichtung (1) nach einem der Ansprüche 3 bis 7;
- eine äußere magensaftresistente Kapsel (9), die die Vorrichtung (1) umschließt.

12. Pille (8) nach Anspruch 10, umfassend:
- ein elektrisch isolierendes Öl (10), das in der äußeren Kapsel (9) umschlossen ist, wobei die Vorrichtung (1) in das elektrisch isolierende Öl (10) eingetaucht ist.

## Revendications

1. Dispositif (1) pour la libération passive et traçable d'une substance dans une solution, comprenant :
- un émetteur (2) configuré pour générer un signal électrique et doté d'une première électrode (3) et d'une seconde électrode (4) ;
- une batterie pouvant alimenter électriquement l'émetteur (2) ;
- un composant à libération passive (5) comprenant ladite substance et qui est dégradable dans une solution afin de libérer ladite substance,
dans lequel
- la première électrode (3) est incorporée dans le composant à libération passive (5),
- le composant à libération passive (5) est constitué au moins en partie d'un matériau électriquement isolant ;
**caractérisé en ce qu'**il comprend un composant électriquement conducteur stable (6) dans lequel la seconde électrode (4) est incorporée.

2. Dispositif (1) selon la revendication 1, dans lequel
- l'émetteur (2) est un oscillateur.

3. Dispositif (1) selon l'une quelconque des revendications précédentes, dans lequel
- l'émetteur (2), la batterie et le composant stable (6) sont des composants électroniques ingérables et de préférence comestibles, et
- le composant à libération passive (5) est une matrice de matière digestible dans laquelle la substance est contenue.

4. Dispositif (1) selon la revendication 3, dans lequel
- le composant à libération passive (5) comprend une matrice oléogel électriquement isolante,
- le composant stable (6) comprend une matrice oléogel électriquement conductrice.

5. Dispositif (1) selon la revendication 4, dans lequel
- la matrice oléogel électriquement isolante comprend de la cire d'abeille et de l'huile de tournesol,
- la matrice oléogel électriquement conductrice comprend de la cire d'abeille, de l'huile de tournesol et du charbon actif.

6. Dispositif (1) selon l'une quelconque des revendications 3 à 5, dans lequel
- la substance comprend au moins un ingrédient actif.

7. Dispositif (1) selon la revendication 6, dans lequel
- l'au moins un ingrédient actif comprend la metformine.

8. Système (100) de surveillance de la libération passive d'une substance dans une solution, comprenant :
- un dispositif (1) selon l'une quelconque des revendications 1 à 7 ;
- un récepteur (7) configuré pour détecter le signal électrique généré par l'émetteur (2) et propagé dans un milieu extérieur aqueux en exploitant la communication intra-corporelle et la commutation de couplage de la communication intra-corporelle.

9. Procédé de surveillance de la libération passive d'une substance dans une solution au moyen d'un système (100) selon la revendication 8, dans lequel le dispositif (1) est un dispositif selon l'une quelconque des revendications 1 à 5, à usage non médical, comprenant les étapes suivantes :
- immerger le dispositif (1) dans une solution ;
- détecter avec le récepteur (7) l'évolution dans le temps du signal électrique généré par l'émetteur (2) du dispositif (1) et propagé dans un milieu aqueux ;
- associer une réduction de l'intensité du signal électrique détecté par le récepteur (7) à l'évolution de l'état de dégradation du composant à libération passive (5) avec la libération de la substance.

10. Procédé selon la revendication 9, comprenant les étapes suivantes :
- identifier la dégradation totale du composant à libération passive (5) lorsque le signal électrique détecté est d'intensité minimale ou négligeable.

11. Pilule (8) pour la libération passive et traçable d'une substance dans l'intestin, comprenant :
- un dispositif (1) selon l'une quelconque des revendications 3 à 7 ;
- une capsule externe gastro-résistante (9) qui renferme le dispositif (1).

12. Pilule (8) selon la revendication 10, comprenant :
- une huile électriquement isolante (10) enfermée dans la capsule extérieure (9), le dispositif (1) étant immergé dans l'huile électriquement isolante (10).
